# EUROPÄISCHE PATENTSCHRIFT

(11) **EP 0 560 177 B1**
(45) Veröffentlichungstag und Bekanntmachung des Hinweises auf die Patenterteilung: **08.01.2003**
(21) Anmeldenummer: 93103301.3
(22) Anmeldetag: 02.03.1993
(51) Int. Cl.: C07D 233/84, C07D 233/86, C07D 233/90, A61K 31/415

(54) **Imidazol-derivate mit Biphenylsulfonylharnstoff- oder Biphenylsulfonylurethan-Seitenkette, Verfahren zu ihrer Herstellung und ihre Verwendung**
Imidazole derivatives having biphenylsulphonylurea or biphenylsulfonylurethane side chains, process for their production and use thereof
Dérivés d'imidazole avec chaînes latérales de biphénylsulphonylurée ou biphénylsulphonyluréthane, procédé pour leur préparation et leur utilisation

(30) Priorität: 07.03.1992 DE 4207241
(43) Veröffentlichungstag der Anmeldung: 15.09.1993
(73) Patentinhaber: HOECHST AKTIENGESELLSCHAFT, 65926 Frankfurt am Main (DE)
(72) Erfinder: Heitsch, Holger, Dr., W-6238 Hofheim/Ts. (DE); Henning, Rainer, Dr., W-6234 Hattersheim/M. (DE); Wagner, Adalbert, Dr., W-6234 Hattersheim/M. (DE); Gerhards, Hermann, Dr., W-6238 Hofheim/Ts. (DE); Becker, Reinhard, Dr., W-6200 Wiesbaden (DE); Schölkens, Bernward, Prof. Dr., W-6233 Kelkheim/Ts. (DE)

(56) Entgegenhaltungen:
- EP-A- 0 324 377
- EP-A- 0 401 030
- EP-A- 0 479 479
- EP-A- 0 503 162

## Beschreibung

Der Entwicklung neuer Angiotensin-II-Rezeptor-Antagonisten wird wachsende Bedeutung im Hinblick auf die Bereitstellung neuer Wirkstoffe beigemessen. Aus EP-A-28834 sind beispielsweise 1-Benzyl-substituierte Imidazolderivate, aus EP-A-253 310 Imidazolderivate mit einer Diarylcarbonsäurefunktion, aus EP-A-324 377 Imidazolderivate mit einer Diaryl-tetrazolyl-Gruppe sowie aus EP-A- 401030 Imidazol derivate mit einer verschiederartig substituierten Diarylgruppe und deren Verwendung als Antagonisten von Angiotensin-II-Rezeptoren bekannt.

In EP-A 0503162 werden ferner 2-n-Butyl-substituierte Imidazol-Derivate, die eine Biphenylsulfonylharnstoff- oder eine -sulfonylurethan-Struktur aufweisen und deren Verwendung als Antagonisten von Angiotensin-II-Rezeptoren vorgestellt.

In der vorliegenden Erfindung werden neue Imidazol-Derivate mit Biphenylsulfonylharnstoff- oder Biphenylsulfonylurethan-Seitenkette beschrieben, die in der Position 2 des Imidazolringes einen speziellen Substituenten R¹ aufweisen und überraschenderweise hochwirksame Angiotensin-II-Rezeptor-Antagonisten in vitro und in vivo sind.

Die Erfindung betrifft Verbindungen der Formel (I), 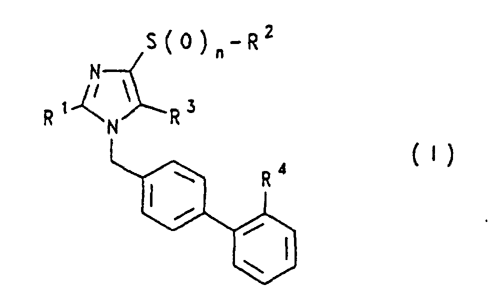 in welcher die Symbole folgende Bedeutung haben:
a)
   - R¹: (C₁-C₃)-Alkyl, vorzugsweise n-Propyl oder Ethyl, insbesondere jedoch n-Propyl
b) R²
   1. (C₁-C₆)-Alkyl, vorzugsweise Methyl;
c) R³
   1. CO-R⁶;
d) R⁴
   1. SO₂-NR⁸-CO-NR⁷R⁹,
   2. SO₂-NH-COO-R⁷, oder
   3. SO₂-NH-CO-R⁷;
e) R⁶
   1. Wasserstoff oder
   2. OR⁷;
f)
   - R⁷,R⁹: gleich oder verschieden
   1. Wasserstoff,
   2. (C₁-C₆)-Alkyl, vorzugsweise Methyl, Ethyl oder Propyl
   3. (C₃-C₆)-Cycloalkyl-(C₁-C₃)-alkyl,
   4. (C₆-C₁₀)-Aryl-(C₁-C₄)-alkyl, vorzugsweise Benzyl
   5. (C₂-C₆)-Alkenyl
g)
   - R⁸: Wasserstoff;
h)
   - n: 0, 1 oder 2;
sowie deren physiologisch verträglichen Salze.

Bevorzugt wird eine Verbindung der Formel (I), in der
- R¹: Ethyl- oder n-Propyl bedeutet, oder deren physiologisch verträglichen Salze. Insbesondere steht R¹ für n-Propyl.

Auch bevorzugt ist eine Verbindung der Formel (I), bei der
- R²: (C₁-C₆)-Alkyl bedeutet,
- R³: COR⁶ bedeutet,
- n: gleich Null ist,
- R⁴: SO₂-NH-CO-OR⁷, SO₂NHCO-NHR⁷ oder SO₂-NH-CO-R⁷ bedeutet,
- R⁶: Wasserstoff oder OR⁷ ist, und
- R⁷: gleich Wasserstoff oder (C₁-C₆)-Alkyl bedeutet, sowie deren physiologisch verträglichen Salze.

Alkyl, Alkenyl und Alkinyl können geradkettig oder verzweigt sein.

Unter Cycloalkyl werden auch Alkyl-substituierte Ringe verstanden.

(C₆-C₁₂)-Aryl ist beispielsweise Phenyl, Naphthyl oder Biphenyl, vorzugsweise Phenyl.

Gegebenenfalls vorkommende Stereozentren können sowohl (R)- als auch (S)-konfiguriert sein.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind.

Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung betrifft auch ein Verfahren zur Herstellung der neuen Verbindungen der Formel (I), sowie deren physiologisch verträglichen Salze, das dadurch gekennzeichnet ist, daß man Verbindungen der Formel (II) 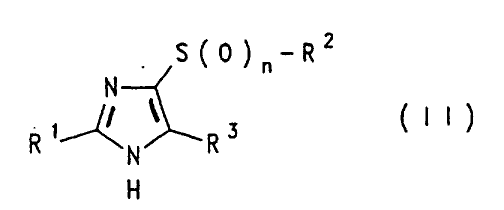 worin R¹, R², R³ und n wie oben definiert sind, alkyliert mit Verbindungen der Formel (III), 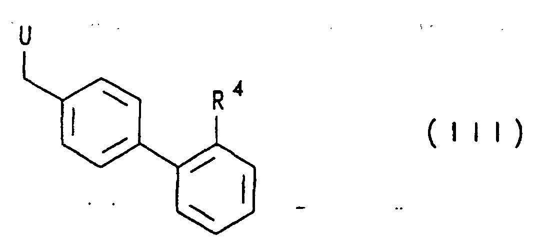 worin R⁴ wie oben definiert sind und U für eine Fluchtgruppe steht, gegebenenfalls temporär eingeführte Schutzgruppen wieder abspaltet, erhaltene Sulfonamide der Formel (I) gegebenenfalls in Urethane der Formel (I) überführt, erhaltene Sulfonamide der Formel (I) gegebenenfalls in Sulfonylharnstoffe der Formel (I) überführt und die erhaltenen Verbindungen der Formel (I) gegebenenfalls in ihre physiologisch verträglichen Salze überführt.

Geeignete Fluchtgruppen U sind vorzugsweise nucleofuge Gruppen (siehe Angew. Chem. 72 [1960] 71) wie Halogen, o-Toluolsulfonat, Mesylat oder Triflat.

Verfahren zur Herstellung der Vorstufen der Formel (II) sind u.a. bekannt aus dem US-Patent 4 355 044 und den oben bereits genannten EP-A-324 377 und EP-A-323841.

Weitere Verfahren werden beschrieben von G.L'abbe (Chem. Rev. 69, 345 (1969)), T. Srodsky ("The Chemistry of the Azido Group", Wiley, New York, 1971, S. 331), H. Wamhoff ("Comprehensive Heterocyclic Chemistry") sowie von S. Katritzky (Ed., Pergamon Press, New York (1984)).

Ein weiteres Verfahren zur Herstellung von Verbindungen der Formel (II) geht von 1-Cyanoglyoxylsäure-2-oxim-Derivaten aus und liefert nach Reduktion des Oxims mit literaturbekannten Reduktionsmitteln und Addition von Mercaptoverbindungen an die Nitrilgruppe unter Verwendung geeigneter Schutzgruppen Vorstufen, die unter Wasser-abspaltenden Bedingungen zu Imidazolen cyclisiert werden können. Für den Cyclisierungsschritt können u.a. Gemische aus PCl₅ und Dimethylaminopyridin (DMAP), POCl₃ und SOCl₂ und deren Gemische mit DMAP verwendet werden.

Zur Alkylierung der Verbindungen der Formel (II) sind z.B. entsprechende Benzylhalogenide, -tosylate, -mesylate oder -triflate oder entsprechende Alkylhalogenide, -tosylate, -mesylate oder -triflate geeignet.

Die Darstellung dieser Verbindungen erfolgt in an sich bekannter Weise, beispielsweise durch Halogenierung der entsprechenden Methylvorstufen. Hierzu wird vorzugsweise N-Bromsuccinimid eingesetzt, siehe z.B. J. Org. Chem. 44, 4733 (1979) und Helv. Chim. Acta 62, 2661 (1979).

Die Biphenylderivate können z.B. ausgehend von Arylboronsäurederivaten durch Kopplung mit substituierten Arylhalogeniden mit Übergangsmetallkatalysatoren, insbesondere Palladium synthetisiert werden. Entsprechende Reaktionen werden von R.B. Miller et al. (Organometallics 1984, 3, 1261) oder von A. Zuzuki et al. (Synthetic Commun. 11 (7), 513 (1981)) beschrieben.

Die Sulfonylurethan-Derivate der Formel (I) können aus entsprechenden Sulfonamiden der Formel (I) durch Umsetzung mit Chlorkohlensäureestern und Basen wie z.B. Kaliumcarbonat in inerten Lösungsmitteln, vorzugsweise bei Temperaturen bis zum Siedepunkt des entsprechenden Lösungsmittels erhalten werden.

Die Sulfonylharnstoff-Derivate der Formel (I) sind wahlweise entweder aus den entsprechenden Sulfonamiden der Formel (I) durch Umsetzung mit Isocyanaten oder mit 2,2,2-Trichloracetamid-Derivaten eines geeigneten Amins in inerten, hochsiedenden Lösungsmitteln wie z.B. DMSO oder aus Sulfonylurethanen der Formel (I) durch Einwirkung des entsprechenden Amins in einem inerten, hochsiedenden Lösungsmittel wie z.B. Toluol bei Temperaturen bis zum Siedepunkt des jeweiligen Lösungsmittels darstellbar.

Analog können Sulfonyl-sulfonamide aus den entsprechenden Sulfonamiden durch Umsetzung mit Sulfonsäurechloriden oder Sulfamoylchloriden hergestellt werden.

Der Sulfonamid-Rest kann, falls erforderlich, ausgehend von einer Aminogruppe mittels Meerwein-Umlagerung hergestellt werden. Hierfür wird das Hydrochlorid des Amins zuerst diazotiert und dann in Gegenwart eines Kupferkatalysators mit Schwefeldioxid in Eisessig umgesetzt. Nachfolgende Einwirkung von Ammoniak führt zur Sulfonamidogruppe.

Die Sulfonamidogruppe wird z.B. temporär durch Überführung in die 2 N,N-Dimethylaminoformylsulfonamidogruppe geschützt. Diese Überführung erfolgt dabei alternativ entweder durch Umsatz der entsprechenden Sulfonamidverbindung mit N, N-Dimethylformamiddimethylacetal oder durch Umsatz der entsprechenden Sulfonamidverbindung mit N,N-Dimethylformamid in Gegenwart von wasserentziehenden Mitteln wie SOCl₃, POCl₃, PCl₅ oder Chlorameisensäureethylestern.

Diese Schutzgruppe kann sowohl unter basischen wie sauren Bedingung abgespalten werden.
Alternativ kann ein entsprechendes Thiophenol durch Oxidation mit Chlor und anschließender Einwirkung von Ammoniak in ein Sulfonamid umgewandelt werden.

Die Alkylierung erfolgt nach prinzipiell bekannten Verfahren in analoger Weise.

Imidazole der Formel (II) werden z.B. in Gegenwart einer Base metalliert. Bevorzugte Basen sind Metallhydride wie Lithium-, Natrium- oder Kaliumhydrid in beispielsweise DMF oder DMSO als Lösungsmittel oder Metallalkoxide der Formel MOR, wobei R für Methyl, Ethyl, t-Butyl steht, und die Reaktion in dem entsprechenden Alkohol, DMF oder DMSO durchgeführt wird. Die so gebildeten Salze der Imidazo-Derivate werden in einem aprotischen Lösungsmittel wie DMF oder DMSO gelöst und mit einer geeigneten Menge Alkylierungsreagenz versetzt.

Eine alternative Möglichkeit zur Deprotonierung der Imidazol-Derivate stellt z.B. die Umsetzung mit Kaliumcarbonat in DMF oder DMSO dar.

Die Oxidation der Thioverbindungen der Formel (I) mit n = O zu den entsprechenden Sulfonen (n = 1) und Sulfoxiden (n = 2) erfolgt bevorzugt durch Persäuren in geeigneten Lösungsmitteln wie z.B. CH₂Cl₂.

Die Umsetzungen werden bei Temperaturen unterhalb Zimmertemperatur bis zum Siedepunkt des Reaktionsgemisches, vorzugsweise zwischen +20°C und dem Siedepunkt des Reaktionsgemisches während etwa 1 bis 10 Stunden durchgeführt.

Die erfindungsgemäßen Verbindungen der Formel (I) haben antagonistische Wirkung auf Angiotensin-II-Rezeptoren und können deshalb beispielsweise zur Behandlung der Angiotensin-II-abhängigen Hypertension verwendet werden. Anwendungsmöglichkeiten bestehen weiterhin bei Herzinsuffizienz, Kardioprotektion, Myocardinfarkt, Herz-Hyperthropie, Arteriosklerose, Nephropathie, Nierenversagen sowie cardiovasculäre Erkrankungen des Gehirns wie transitorischen ischämischen Attacken und Gehirnschlag.

Renin ist ein proteolytisches Enzym aus der Klasse der Aspartyl-Proteasen, welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Antiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt.

Postrezeptor-Wirkungen sind unter anderem Stimulation des Phosphoinositol-Umsatzes (Ca²⁺-Freisetzung), Aktivierung der Proteinkinase C und Facilitation von c-AMP abhängigen Hormon-Rezeptoren.

Die Affinität der Verbindungen der Formel (I) zum Angiotensin-II-Rezeptor kann durch Messung der ¹²⁵J-Angiotensin-II- oder ³H-Angiotensin-II-Verdrängung von Rezeptoren an Membranen der zona glomerulosa von Rindernebennieren bestimmt werden. Dazu werden die präparierten Membranen in Puffer bei pH 7.4 suspendiert.

Um die Degradierung des Radioliganden während der Inkubierung zu verhindern, wird Aprotinin, ein Peptidase Inhibitor, zugesetzt. Zusätzlich werden ca. 14.000 cpm eines Tracers mit spezifischer Aktivität von 74 TBq/mmol (käuflich bei Amersham Buchler, Braunschweig, BRD) und eine Menge Rezeptorprotein, die 50 % des Tracers bindet, verwendet. Die Reaktion wird durch Zugabe von 50 µl Membransuspension zu einer Mischung von 100 µl Puffer + Aprotinin; 50 µl Puffer mit oder ohne Angiotensin-II oder Rezeptorenantagonist und 50 µl Tracer gestartet. Nach einer Inkubationszeit von 60 Minuten bei einer Temperatur von 25°C werden gebundener und freier Radioligand durch einen Filtrationsassay (z.B. mit Whatman® GFIC Filtern auf einem Skatron®-Zellsammler) getrennt.

Unspezifische Bindungen werden durch Behandlung der Filter mit 0,3 % Polyethylenimin pH=10 verhindert (z.B. Sigma, Nr. 3143). Durch Messung der Radioaktivität in einem Gamma-Szintillationszähler wird die Stärke der Verdrängung des Radioliganden vom Rezeptor bestimmt.

Die IC₅₀-Werte, welche die Konzentration des Inhibitors bedeuten, um 50 % des Uganden zu verdrängen, werden nach J. Theor. Biol. 59, 253 (1970) bestimmt. Sie liegen für die Verbindungen der Formel (I) im Bereich von 1•10⁻⁴ bis 1•10⁻⁹ M.

Alternativ kann die Affinität der Verbindungen der Formel (I) zum Angiotensin-II-Rezeptor durch Messung der ¹²⁵J-Angiotensin-II oder ³H-Angiotensin-II-Verdrängung von Rezeptorpräparationen aus verschiedenen Organen (Leber, Lunge, Nebenniere, Hirn etc.) bestimmt werden.

Dazu werden die präparierten Membranen in einem Inkubationspuffer (z.B. 20 mM Tris, pH 7.4, enthaltend 135 mM NaCI, 10 mM KCI, 10 mM MgCl₂, 5 mM Glucose, 0.2 % Rinderserumalbumin sowie die Proteaseinhibitoren PMSF 0,3 mM und Bacitracin 0,1 mM) suspendiert und zusammen mit dem radioaktiv markierten Angiotensin-II und verschiedenen Konzentrationen der zu testenden Verbindungen für 90 min bei 25°C inkubiert. Anschließend werden gebundener und freier Radioligand durch Filtration über Microglasfaserfilter (z.B. GF51, Schleicher & Schüll auf einem Zellsammler (SKATRON)) getrennt.

Durch Messung der Rezeptor-gebundenen Radioaktivität auf den Filtern mittels eines Beta- oder Gamma-Spektrometers wird der Grad der Verdrängung des Radioliganden vom Rezeptor durch die Testverbindungen bestimmt. Die Stärke der Verdrängung des Radioliganden vom Rezeptor durch die Testverbindungen wird mit der IC₅₀ angegeben, d.i. die Konzentration des Inhibitors, die 50 % des gebundenen Radioliganden vom Rezeptor verdrängt. Die Berechnung der IC₅₀-Werte erfolgt mittels einer PC-Software (z.B. LIGAND, G.A. McPherson 1985, Elsevier-BIOSOFT, 68 Hills Road, Cambridge CB 2 1LA, U.K.). Die für Verbindungen der Formel (I) gemessenen IC₅₀-Werte liegen im Bereich von 1 x 10⁻⁵ bis 1 x 10⁻¹¹ M.

Zur Bestimmung der antagonistischen Wirkung der Verbindungen der Formel (I) in vivo kann ihr inhibitierender Effekt auf den Angiotensin-II-induzierten Blutdruckanstieg an entmarkten Sprague-Dawley-Ratten (Möllegard, Dänemark) gemessen werden. Die intravenöse Applikation erfolgt in die Penis-Vene.

Nach Präparation des Tieres und 20-minütiger Wartezeit zur Stabilisierung der hämodynamischen Parameter werden 3 aufeinanderfolgende Injektionen von 10, 30 und 100 mg Angiotensin-II in 0,1 ml wäßriger Lösung in 5-Minuten-Intervallen verabreicht. Die Verbindungen der Formel (I) werden in destilliertem Wasser, eventuell unter 10%igem Ethanol- und/oder Basen-(pH < 10) oder Säuren -(pH > 3)-Zusatz, gelöst und in Dosen von 1-300 µg/kg intravenös bzw. 5-1000 µg/kg intraduodenal appliziert.

Bei intraduodenaler Gabe erfolgt die Angiotensin-II-Injektion nach 20, 40 und 60 Minuten, während bei intravenöser Gabe die "pressor response sequence" in 10 Minuten-Intervallen erfolgt.

Die Verbindungen der Formel (I) sind insbesondere im Bereich von 1-300µg/kg intravenös bzw. 5-300 µg/kg intraduodenal wirksam.

Die Erfindung bezieht sich ebenso auf pharmazeutische Zubereitungen bestehend im wesentlichen aus einer Verbindung der Formel (I) und gegebenenfalls anderen Wirkstoffen, wie z.B. Diuretika oder nichtsteroidalen anti-inflammatorischen Wirkstoffen. Die Verbindungen der Formel (I) können auch als Diagnostika für das Renin-Angiotensin-System verwendet werden.

Pharmazeutische Präparate enthalten eine wirksame Menge des Wirkstoffs der Formel (I) und eventuell andere Wirkstoffe zusammen mit einem anorganischen oder organischen pharmazeutisch verwendbaren Trägerstoff und gegebenenfalls weitere Zusatz- oder Hilfsstoffe. Die Anwendung kann intraanasal, intravenös, subkutan oder peroral erfolgen. Die Dosierung des Wirkstoffs hängt von der Warmblüter-Spezies, dem Körpergewicht, dem Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen wie Tabletten, Dragees, Steckkapseln, wäßrige, alkoholische oder ölige Suspensionen oder wäßrige, alkoholische oder ölige Lösungen gebracht. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke, verwendet werden. Dabei kann die Zubereitung als Trocken- oder Feuchtgranulat erfolgen. Als ölige Trägerstoffe oder Lösungsmittel kommen beispielsweise pflanzliche oder tierische Öle, wie Sonnenblumenöl und Lebertran in Betracht.

Zur subkutanen oder intravenösen Applikation werden die aktiven Verbindungen oder deren physiologisch verträgliche Salze, gegebenenfalls mit den dafür üblichen Substanzen wie Lösungsvermittlern, Emulgatoren oder weiteren Hilfsstoffen, in Lösungen, Suspensionen oder Emulsionen gebracht. Als Lösungsmittel kommen z.B. Wasser, physiologische Kochsalzlösung oder Alkohole, wie Ethanol, Propandiol oder Glycerin, sowie Zuckerlösungen wie Glucose- oder Mannitlösungen oder Mischungen aus den genannten Lösungsmitteln in Frage.

### Liste der Abkürzungen:

- Ac: Acetyl
- DMF: N,N-Dimethylformamid
- DME: Dimethoxyethan
- EE: Ethylacetat
- DCI: Desorption-Chemical Ionisation
- FAB: Fast Atom Bombardment
- RT: Raumtemperatur
- mp: Schmelzpunkt
- h: Stunde(n)
- Min.: Minute(n)

Die Erfindung wird durch die nachfolgenden Beispiele erläutert:

### Beispiel 1

1-[(2'-n-Propylaminocarbonylaminosulfonyl-biphenyl-4-yl)methyl]-2-n-prppyl-4-methylthio-imidazol-5-carbonsäureethylester 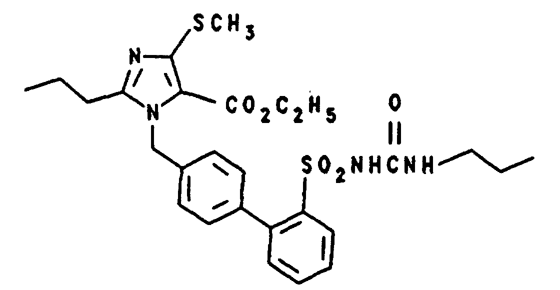
a) 2-Amino-2-cyano-essigsäureethylester
   Zu 70 g (0,492 mol) 2-Cyanoglyoxylsäurethylester-2-oxim in 700 ml Wasser und 560 ml gesättigte NaHCO₃-Lösung werden bei RT portionsweise innerhalb von 20 Min. 228 g (1,3 mol) Natriumdithionit gegeben. Es wird 12 h bei 35°C gerührt und nach Abkühlen und Sättigung mit NaCI mit CH₂Cl₂ extrahiert. Trocknung mit Na₂SO₄ und Einengung zur Trocknung liefert 30 g der Titelverbindung als Öl
   Rf (CH₂Cl₂/CH₃OH 9/1) = 0,6.
b) 2-Cyano-2-n-proplycarbonylaminoessigsäureethylester
   Zu 30 g (0,233 mol) der Verbindung aus 1a) in 250 ml abs. CH₂Cl₂ und 18,9 ml (0,233 mol) Pyridin tropft man bei 0-5°C eine Lösung von 24,2 ml (0,23 mol) Buttersäurechlorid in 25 ml CH₂Cl₂ zu. Anschließend wird 12 h bei RT gerührt. Die organische Phase wird 3 x mit H₂O und 1 x mit gesättigter NaCl-Lösung gewaschen, mit Na₂SO₄ getrocknet und eingeengt. Kristallisation aus Diisopropylether liefert 29,5 g der Titelverbindung
   Rf (CH₂Cl₂/CH₃OH 9/1) = 0,7
   mp: 106°C.
c) 3-Amino-2-n-propylcarbonylamino-3-methylthioacrylsäureethylester
   zu 29,5 g (0,149 mol) der Verbindung aus 1b) und 2,05 ml (0,0145 mol) Triethylamin in 500 ml Ethanol gibt man bei RT 14,3 g (0,297 mol) kondensiertes Methylmercaptan. Nach 4 Tagen Stehenlassen bei RT wird das Lösungsmittel entfernt und der Rückstand aus Diisopropylether kristallisiert, wobei 36,2 g der Titelverbindung resultieren.
   Rf (CH₂CL₂/CH₃OH 9:1) = 0,4
   mp: 119°C
d) 2-n-Propyl-4-methylthio-imidazol-5-carbonsäureethylester
   zu 29,16 g (0,142 mol) Phosphorpentachlorid in 240 ml CH₂Cl₂ tropft man bei
   -78°C 19,3 g (0,157 mol) 4-Dimethylaminopyridin in 120 ml CH₂Cl₂. Nach 10 Min. werden 17,6 g (0,071 mol) der Verbindung aus 1c) in 200 ml CH₂Cl₂ zugetropft. Es wird auf RT erwärmt und
   2,5 h bei RT gerührt. Unter Eiskühlung werden anschließend 1,6 I 1N NaHCO₃-Lösung zugesetzt, 1 h gerührt und über Nacht stehengelassen. Nach Trennung der Phasen wird die wäßrige Phase 3 x mit EE extrahiert, die vereinten organischen Phasen mit Na₂SO₄ getrocknet und eingeengt. Chromatographie an SiO₂ mit CH₂Cl₂/EE 4/1 liefert 5,6 g der Titelverbindung als Öl.
   Rf (CH₂Cl₂/EE 4:1) = 0,4
   MS (DCI) : 229 (M+H).
e) Sulfonamidobrombenzol
   51,6 g (0,3 mol) o-Bromanilin werden unter Argon-Atmosphäre zu einer Lösung aus 100 ml konz. HCI und 30 ml Eisessig gegeben, bei -10°C eine Lösung von 22,4 g Natriumnitrit in 30 ml Wasser zugetropft und die Reaktionslösung 60 Min. bei -5°C gerührt. Die erhaltene Lösung wird zu einer mit SO₂ gesättigten Lösung von 7 g CuCl₂ x 2H₂O und 0,5 g CuCI in 300 ml Eisessig getropft, die Mischung nach 60 Min. Rühren bei Raumtemperatur in ein Eis/Wasser-Gemisch gegossen, mit Ether extrahiert, die Ether-Extrakte mit ges. NaHCO₃-Lösung und Wasser gewaschen, über MgSO₄ getrocknet und eingeengt. Die erhaltenen 67,8 g Sulfonylchloridverbindung werden in 500 ml Aceton unter Kühlung mit 300 ml konz. Ammoniak versetzt. Nach Abzug des Acetons wird die resultierende Suspension mit Wasser verdünnt, die ausfallenden weißen Kristalle abgesaugt, mit H₂O gewaschen und im Hochvakuum getrocknet. Die Titelverbindung wird ohne weitere Reinigung in der folgenden Reaktion eingesetzt.
   mp: 190°C.
f) 2-N,N-Dimethylaminoformylsulfonamidobrombenzol
   0,236 mol der Verbindung aus Beispiel 1e) werden in 150 ml abs. DMF mit 40 ml N,N-Dimethylformamiddimethylacetal 2 h bei Raumtemperatur gerührt. Die Reaktionslösung wird auf 200 ml 5%ige NaHSO₄-Lösung/Eis (1:1) gegossen, der ausfallende Niederschlag abgesaugt, mit H₂O gewaschen und im Vakuum getrocknet. Man erhält 67 g der Titelverbindung.
   mp: 148°C,
   Rf (SiO₂), EE/Heptan 1:1) = 0,1
   MS (DCI) : 291/293 (M + H)
g) 4'-Methyl-biphenyl-2-N,N-dimethylaminoformylsulfonamid Zu 11 g (37,9 mmol) der Verbindung aus Beispiel 1f), 1 g Triphenylphosphin, 8 g Na₂CO₃ in 150 ml Toluol und 40 ml H₂O gibt man unter Argon zuerst 420 mg Pd(OAc)₂ und anschließend 5,66 g (41,9 mmol) Tolylboronsäure in 100 ml Ethanol. Nun wird 4 h zum Sieden erhitzt, dann eingeengt und in 500 ml EE und 500 ml H₂O aufgenommen. Der entstehende Niederschlag wird abfiltriert und als Titelverbindung charakterisiert. Die EE-Phase wird abgetrennt, über Na₂SO₄ getrocknet und eingeengt. Chromatographie an SiO₂ mit EE liefert einen weiteren Anteil der Titelverbindung;
   Gesamtausbeute: 7,6 g
   mp: 181-184°C
   Rf (SiO₂, EE/Heptan 1:1) = 0,2
   MS (DCI): 303 (M+H)
h) 4'-Brommethylbiphenyl-2-N,N-dimethylaminoformylsulfonamid
   7,4 g (0,025 mol) der Verbindung aus 1g) werden mit 4,6 g (0,026 mol) N-Bromsuccinimid in 130 ml Chlorbenzoin Gegenwart von 300 mg Benzoylperoxid 2 h zum Rückfluß erhitzt. Nach dem Abkühlen werden 50 ml gesättigte Na₂SO₃-Lösung zugesetzt, die organische Phase abgetrennt, mit gesättigter Na₂CO₃-Lösung, Wasser und gesättigter NaCl-Lösung gewaschen, über Na₂SO₄ getrocknet und eingeengt. Der Rückstand wird mit EE verrührt und 6,7 g der Titelverbindung abgesaugt.
   mp: 168-171°C
   Rf(SiO₂, EE) = 0,5
   Ms (DCI) 381/383 (M + H)
i) 1-[(2'-N,N-Dimethylaminoformylsulfonamido-biphenyl-4-yl]methyl]-2-n-propyl-4-methylthio-imidazol-5-carbonsäureethylester
   2,0 g (8,75 mmol) der Verbindung aus 1d), 4,15 g (8,75 mmol) der Verbindung aus 1h) (75%ig) und 1,25 g (9,0 mmol) K₂CO₃ werden in 50 ml abs. DMF über Nacht bei RT gerührt. Es wird eingeengt, der Rückstand in 400 ml EE gelöst, die EE-Lösung 3 x mit Wasser gewaschen, über Na₂SO₄ getrocknet und eingeengt. Der Rückstand wird mit Ethanol und Diisopropylether verrührt und 4,14 g der Titelverbindung als ausfallender Niederschlag abgesaugt.
   mp: 169-171°C
   Rf (SiO₂, EE) = 0,4
   MS (FAB) : 529 (M + H)
j) 1-[(2'-Sulfonamidobiphenyl-4-yl)methyl]-2-n-propyl-4-methylthioimidazol-5-carbonsäureethylester
   4 g (7,60 mmol) der Verbindung aus 1i) werden in 40 ml Methanol mit 20 ml konz. Salzsäure 3 h unter Rückfluß gekocht. Man läßt auf RT abkühlen, destilliert das Lösungsmittel ab, stellt den pH der wäßrigen Lösung mit 6 N NaOH-Lösung auf 5-6 ein und extrahiert mehrmals mit EE. Die vereinten EE-Phasen werden über Na₂SO₄ getrocknet und eingeengt. Der resultierende Schaum wird mit Ethanol und Diisopropylether verrührt und der Niederschlag abgesaugt. Es resultieren 3 g der Titelverbindung.
   mp: 125-127°C
   Rf (SiO₂, EE) = 0,7
   MS (FAB) : 474 (M + H)
k) 1-[(2'-n-Propylaminocarbonylaminosulfonyl-biphenyl-4-yl)methyl]-2-n-propyl-4-methylthio-imidazol-5-carbonsäureethylester
   2 g (4,22 mmol) der Verbindung aus 1j) werden in 50 ml abs. Aceton mit 1,75 g (12,66 mmol) wasserfreiem K₂CO₃ versetzt. Nach 30 Min. Erhitzen zum Rückfluß wird diese Lösung mit 395 µl (4,22 mmol) Propylisocyanat versetzt und 1 h am Rückfluß gerührt. Anschließend läßt man abkühlen, versetzt mit 15 ml 2N HCI, engt am Vakuum ein und extrahiert mehrfach mit CH₂Cl₂. Trocknung über Na₂SO₄, Einengung und Kristallisation aus EE liefert 1,9 g der Titelverbindung.
   mP: 160-165°C
   Rf(SiO₂, EE/Heptan 2/1) = 0,25
   MS (FAB) : 559 (M+H)

### Beispiel 2

1-[(2'-n-Propylaminocarbonylaminosulfonyl-biphenyl-4-yl)methyl]-2-n-propyl-4-methylthio-imidazol-5-carbonsäure

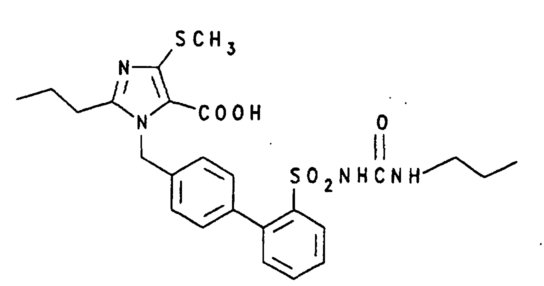

700 mg (1,25 mmol) der Verbindung aus 1k) werden in 40 ml Methanol mit 10 ml 2 N NaOH-Lösung 3 Tage bei RT gerührt. Das Lösungsmittel wird anschließend im Vakuum entfernt, die wäßrige Lösung mit 2 N Salzsäure auf pH=6 eingestellt, der ausfallende Niederschlag abgesaugt und im Hochvakuum getrocknet. Es resultieren 600 mg der Titelverbindung
mp: 133-135°C
Rf (SiO₂, CH₂Cl₂/Methanol 10/1) = 0,19
MS (FAB) : 531 (M+H)

### Beispiel 3

1-[(2'-Ethoxycarbonylaminosulfonyl-biphenyl-4-yl)methyl]-2-npropyl-4-methylthio-imidazol-5-carbonsäureethylester

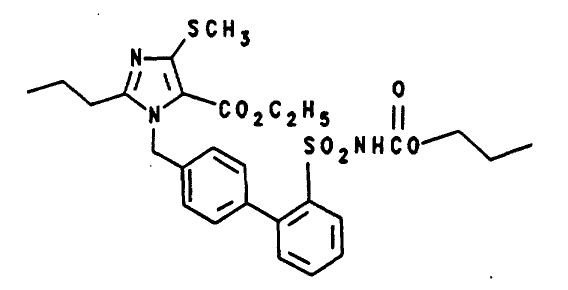

1,5 g (3,17 mmol) der Verbindung aus 1j) werden unter Argon-Atmosphäre in 25 ml abs. DME mit 0,876 g (6,34 mmol) K₂CO₃ und 0,35 ml (3,17 mmol) Chlorameisensäureethylester 3 h am Rückfluß gekocht. Anschließend wird das Lösungsmittel weitestgehend abgezogen, der pH-Wert der verbleibenden Lösung mit 10%iger KH₂PO₄-Lösung auf etwa 4 eingestellt und mehrfach mit EE extrahiert. Die vereinten EE-Extrakte werden mit ges. NaCl-Lösung gewaschen, über MgSO₄ getrocknet, eingeengt und der Rückstand im Hochvakuum getrocknet. Man erhält, 1,79 g der Titelverbindung als gelben Schaum.
Rf (SiO₂, EE) = 0,5
MS (FAB) : 546 (M + H)

### Beispiel 4

1-[(2'-Ethoxycarbonylaminosulfonyl-biphenyl-4-yl)methyl]-2-npropyl-4-methylthio-imidazol-5-carbonsäure

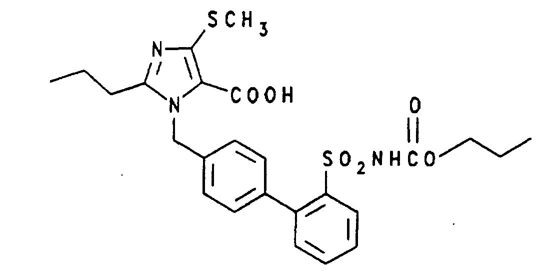

500 mg (0,92 mmol) der Verbindung aus Beispiel 3) werden in 3 ml Ethanol mit 2,5 ml 2 N NaOH-Lösung 24 h bei RT gerührt. Das Lösungsmittel wird im Vakuum entfernt, die verbleibende wäßrige Lösung mit 2 N Salzsäure auf etwa pH 5 eingestellt und der ausfallende Niederschlag abgesaugt. Man erhält 380 mg der Titelverbindung in Form eines schwach gelbgefärbten Festproduktes.
mp: 156-160°C,
Rf (SiO₂, CH₂Cl₂/CH₃OH 9/1) = 0,3
MS (FAB) : 518 (M+H)

### Beispiel 5

1-[(2'-Methylaminocarbonylaminosulfonyl-biphenyl-4-yl]methyl]-2-ethyl-4-methylthioimidazol-5-carbonsäureethylester 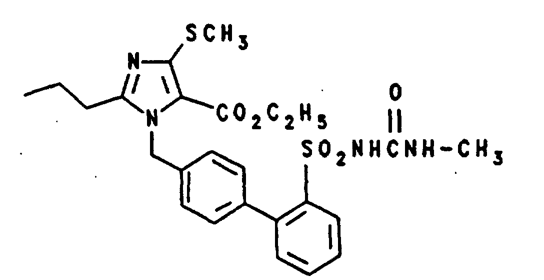
a) 2-Cyano-2-ethylcarbonylaminoessigsäureethylester
   Die Titelverbindung wird nach dem in Beispiel 1b) angegebenen Verfahren hergestellt, wobei hier statt Buttersäurechlorid Propionsäurechlorid mit der Verbindung aus 1a) umgesetzt wird. Es resultieren aus 12,8 g (0,1 mol) der Verbindung aus 1a) 11,4 g der Titelverbindung.
   mp: 111-113°C,
   Rf (SiO₂, EE) = 0,6
   MS (DCI) : 185 (M + H)
b) 3-Amino-2-ethylcarbonylamino-3-methylthioacrylsäureethylester
   Diese Verbindung wird analog zu dem in Beispiel 1c) angegebenen Verfahren hergestellt.
   mp: 127°C,
   Rf (SiO₂, EE) = 0,18
   MS (DCI) : 233 (M + H)
c) 2-Ethyl-4-methylthio-imidazol-5-carbonsäureethylester
   Die Herstellung dieser Verbindung erfolgt analog zu dem in 1d) angeführten Verfahren.
   mp: 141-143°C,
   Rf (SiO₂, EE) = 0,4
   MS (DCI) : 215 (M+H)
d) 1-[(2'-N,N-Dimethylaminoformylsulfonamido-biphenyl-4-yl)-methyl]-2-ethyl-4-methylthio-imidazol-5-carbonsäureethylester
   Die Titelverbindung wird aus Verbindung 5c) und Verbindung 1h) nach dem in Beispiel 1i) beschriebenen Verfahren hergestellt.
   mp: 189-194°C
   Rf (SiO₂, EE) = 0,3
   MS (FAB) : 515 (M+H)
e) 1-[(2'-Sulfonamidobiphenyl-4-yl)methyl]-2-ethyl-4-methylthio-imidazol-5-carbonsäureethylester
   Die Titelverbindung wird nach dem Verfahren des Beispiels 1j) aus der Verbindung aus Beispiel 5d) hergestellt.
   mp: 153-155°C
   Rf (SiO₂, EE) = 0,5
   MS (FAB) : 460 (M+H)
f) 2,2,2-Trichloro-N-methyl-acetamid
   1,6 g (51,5 mmol) Methylamin werden kondensiert und in 20 ml abs. Dioxan mit 7,14 ml (51,5 mmol) Triethylamin und 5,7 ml (51,5 mmol) Trichloracetylchlorid, gelöst in 10 ml abs. Dioxan, versetzt und die resultierende Lösung 3 h bei RT gerührt. Anschließend wird mit Wasser versetzt, der pH-Wert der Lösung mit 2 N Salzsäure auf etwa 1 eingestellt und die ausfallende Titelverbindung (7,6 g) abgesaugt.
   mp : 90-95°C.
g) 1-[(2'-Methylaminocarbonylaminosulfonyl-biphenyl-4-yl)-methyl]-2-ethyl-4-methylthio-imidazol-5-carbonsäureethylester
   135 mg (0,316 mmol) der Verbindung aus 5e) werden in 2 ml abs. DMSO unter Argon-Atmosphäre mit 38 mg (0,1 mmol) gepulvertem NaOH und 61 mg (0,348 mmol) der Verbindung aus 5f) 30 Min. bei 80°C gerührt. Die Reaktionslösung wird auf Eiswasser gegossen, mit 2 N Salzsäure angesäuert und mehrfach mit EE extrahiert. Nach Waschen der vereinten EE-Phasen mit gesättigter NaCl-Lösung, Trocknung über MgSO₄ und Einengung wird der erhaltene kristalline Rückstand mit wenig EE verrührt. Absaugen des Niederschlags liefert 97 mg der Titelverbindung.
   mp: 220-223°C
   Rf (SiO₂, CH₂Cl₂/CH₃OH 9/1) = 0,6
   MS (FAB) : 517 (M+H)

### Beispiel 6

1-[(2'-Methylaminocarbonylaminosulfonyl-biphenyl-4-yl)-methyl]-2-ethyl-4-methylthio-imidazol-5-carbonsäure

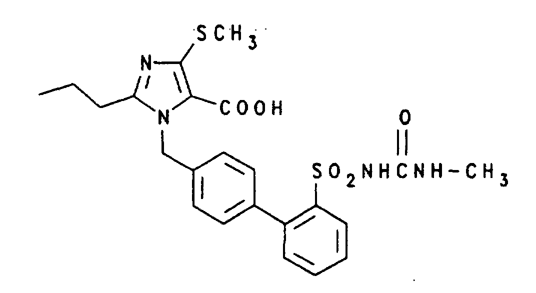

Die Titelverbindung wird nach dem Verfahren des Beispiels 2) aus der Verbindung aus 5g) hergestellt. Aus 50 mg (0,1 mmol) der Verbindung 5g) resultieren 40,5 mg der Titelverbindung.
mp: 155°C
Rf (SiO₂, CH₂Cl₂/CH₃OH/Essigsäure 9/1/0,2) = 0,46
MS (FAB) : 489 (M+H)

### Beispiel 7

1-[(2'-Ethoxycarbonylaminosulfonyl-biphenyl-4-yl)methyl]-2-ethyl-4-methylthio-imidazol-5-carbonsäureethylester

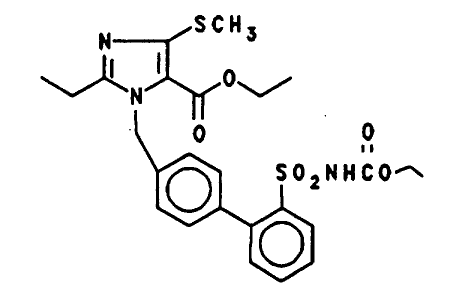

1,4 g (3 mmol) der Verbindung aus Beispiel 5 e) und 825 mg (6 mmol) K₂CO₃ werden in 25 ml abs. DME mit 0,3 ml (3,05 mmol) Chlorameisensäureethylester 3 Stunden am Rückfluß gekocht. Es wird eingeengt, mit 10%iger KH₂PO₄-Lösung auf pH ∼5 eingestellt und mit EE extrahiert. Nach Trocknung über Na₂SO₄ und Einengung resultieren 1,45 g der Titelverbindung.
Rf (SiO₂), EE) = 0,3
MS (FAB): 532 (M+H)

### Beispiel 8

1-[(2'-n-Propylamino-carbonylaminosulfonyl-biphenyl-4-yl)methyl]-2-ethyl-4-methylthio-imidazol-5-carbonsäureethylester

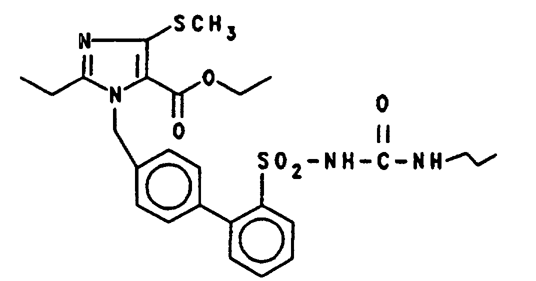

300 mg (0,56 mmol) der Verbindung aus Beispiel 7 werden in 7 ml abs. Toluol mit 1,5 ml n-Propylamin 3 Stunden unter Rückfluß gekocht. Nach Einengung und Chromatographie an SiO₂ mit EE als Laufmittel resultieren 130 mg der Titelverbindung.
m.p.: 202-203°C,
Rf (SiO₂), EE) = 0,24
MS (FAB): 545 (M+H)

### Beispiel 9

1-[(2'-n-Propylaminocarbonylaminosulfonyl-biphenyl-4-yl)methyl]-2-ethyl-4-methylthio-imidazol-5-carbonsäure

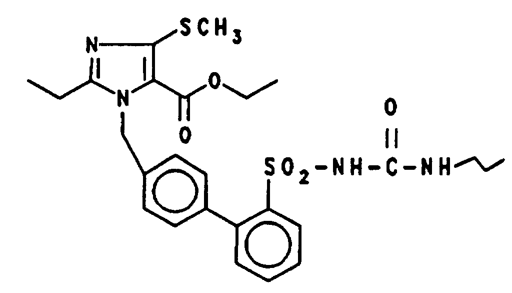

Die Titelverbindung wird nach dem Verfahren des Beispiels 2) aus der Verbindung aus Beispiel 8) hergestellt. Aus 100 mg (0,18 mmol) der Verbindung 8) resultieren 70 mg der Titelverbindung.
m.p.: 135-140°C,
Rf (SiO₂), EE) = 0,1
MS (FAB): 517 (M + M)

### Beispiel 10

1-[(2'-n-Propylaminocarbonylaminosulfonyl-biphenyl-4-yl)methyl]-2-n-propyl-4-methylsulfonyl-imidazol-5-carbonsäureethylester

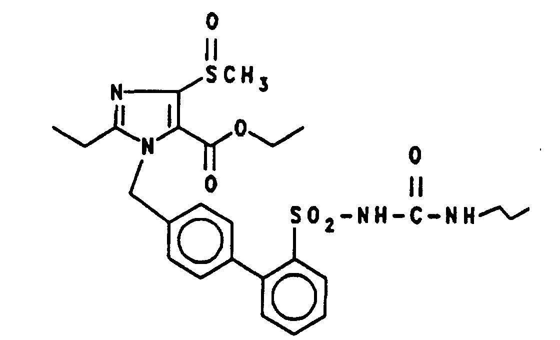

95,0 mg (0,17 mmol) der Verbindung aus Beispiel 1) werden in 10 ml abs. CH₂Cl₂ bei -78°C mit 59 mg (0,17 mmol) m-Chlorperbenzoesäure (50%ig) 20 Minuten gerührt. Es wird mit 10 ml 10%iger Natriumbisulfit-Lösung versetzt, auf Raumtemperatur erwärmt und nach Phasentrennung mit EE extrahiert. Die vereinten organischen Phasen werden mit ges. Na₂CO₃-Lösung gewaschen, über Na₂SO₄ getrocknet und eingeengt. Es resultieren 110 mg der Titelverbindung. m.p.: 65-68°C,
Rf (SiO₂), EE) = 0,1
MS (FAB): 575 (M+M)

### Beispiel 11

1-[(2'-n-Propylaminocarbonylaminosulfonyl-biphenyl-4-yl)methyl]-2-n-propyl-4-methylsulfoxyl-imidazol-5-carbonsäure

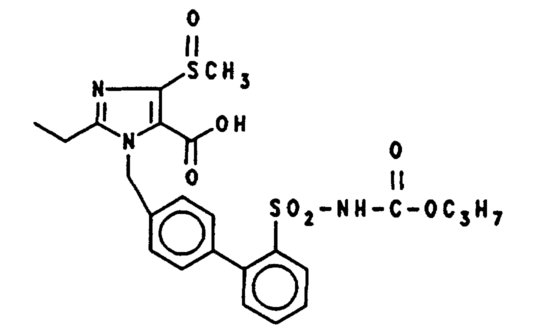

Die Titelverbindung resultiert aus der Verbindung des Beispiels 10) nach dem Verfahren des Beispiels 2). Aus 100 mg (0,17 mmol) der Verbindung aus Beispiel 10) erhält man 83 mg der Titelverbindung.
m.p.: 105-108°C,
Rf (SiO₂), EE) = 0,1
MS (FAB): 547 (M+M)

### Beispiel 12

1-[(2'-Ethoxycarbonylaminosulfonyl-biphenyl-4-yl)methyl]-2-n-propyl-4-methylsulfonyl-imidazol-5-carbonsäureethylester

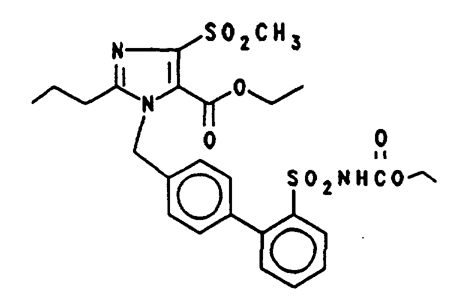

350 mg (0,64 mmol) der Verbindung aus Beispiel 3) werden mit 443 mg (1,28 mmol) m-Chlorbenzoesäure (50%ig) in 20 ml abs. CH₂Cl₂ 1 Stunde am Rückfluß erhitzt. Die Aufarbeitung erfolgt analog zu der des Beispiels 10) und liefert 364 mg der Titelverbindung als farblosen Schaum.
Rf (SiO₂), CH₂Cl₂ (CH₃OH 9:1) 0,74, MS (FAB): 578 (M + H)

### Beispiel 13

1-[(2'-Ethoxycarbonylaminosulfonyl-biphenyl-4-yl)methyl]-2-n-propyl-4-methylsulfonyl-imidazol-5-carbonsäure

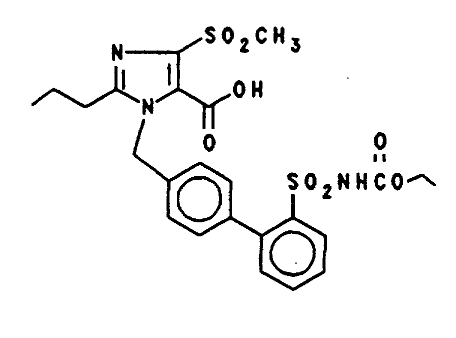

Aus 120 mg (0,2 mmol) der Verbindung aus Beispiel 12) resultieren nach dem Verfahren des Beispiels 2) 84 mg der Titelverbindung.
mp.: 156-159°C,
Rf (SiO₂), CH₂Cl₂ (CH₃OH 9:1:0,2) = 0,5,
MS (FAB): 550 (M + H)

### Beispiel 14

1-[(2'-Ethylaminocarbonylaminosulfonyl-biphenyl-4-yl)methyl]-2-n-propyl-4-methylsulfonyl-imidazol-5-carbonsäureethylester

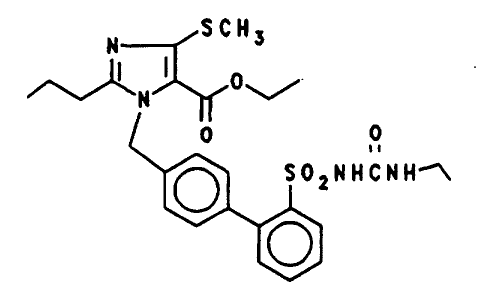

Der Umsatz von 200 mg (0,42 mmol) der Verbindung aus Beispiel 1j) mit 34 µl (0,42 mmol) Ethylisocyanat nach dem Verfahren des Beispiels 1 k) liefert 170 mg der Titelverbindung.
mp.: 161-162°C,
Rf (SiO₂, EE) = 0,43, MS (FAB): 545 (M+H)

### Beispiel 15

1-[(2'-Ethylaminocarbonylaminosulfonyl-biphenyl-4-yl)methyl]-2-n-propyl-4-methylsulfonyl-imidazol-5-carbonsäure

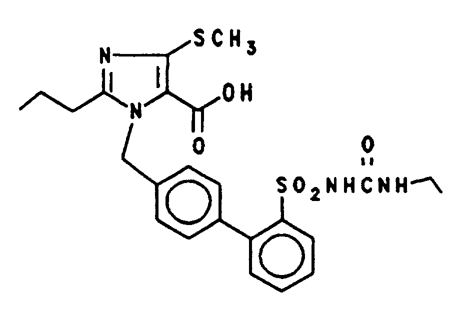

Aus 61 mg (0,11 mmol) der Verbindung aus Beispiel 14) resultieren nach dem Verfahren des Beispiels 2) 56 mg der Titelverbindung.
mp.: 131°C,
Rf (SiO₂, CH₂Cl₂, CH₃OH 10:1) = 0,2,
MS (FAB): 517 (M+H)

### Beispiel 16

1-[(2'-Allylaminocarbonylaminosulfonyl-biphenyl-4-yl)methyl]-2-n-propyl-4-methylthio-imidazol-5-carbonsäureethylester

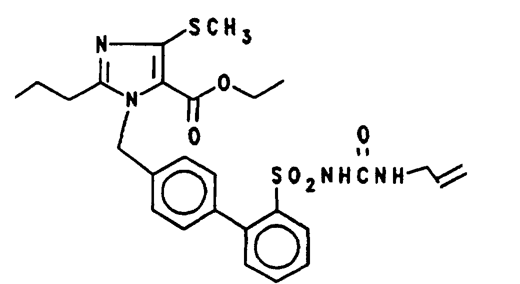

Der Umsatz von 200 mg (0,42 mmol) der Verbindung aus Beispiel 1 j) mit 38 µl (0,42 mmol) Allylisocyanat nach dem Verfahren des Beispiels 1 k) liefert 150 mg der Titelverbindung.
mp.: 184°C,
Rf (SiO₂, EE) : 0,43,
MS (FAB): 557 (M+H)

### Beispiel 17

1-[(2'-Allylaminocarbonylaminosulfonyl-biphenyl-4-yl)methyl]-2-n-propyl-4-methylthio-imidazol-5-carbonsäure

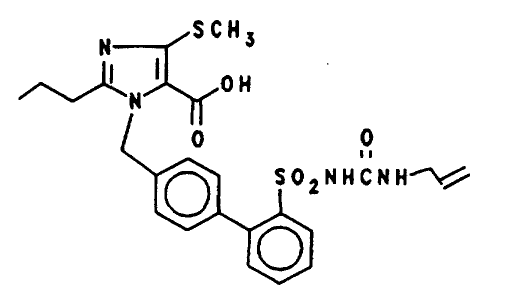

Aus 60 mg (0,1 mmol) der Verbindung aus Beispiel 16) resultieren nach dem Verfahren des Beispiels 2) 54 mg der Titelverbindung.
mp.: 148°C,
Rf (SiO₂, CH₂Cl₂/CH₃OH 10:1): 0,3,
MS (FAB): 529 (M+H)

### Beispiel 18

1-[(2'-Methoxycarbonylaminosulfonyl-biphenyl-4-yl)methyl]-2-n-propyl-4-methylthio-imidazol-5-carbonsäureethylester 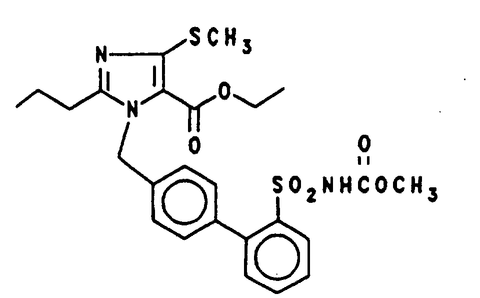 500 mg (1,06 mmol) der Verbindung aus Beispiel 1 j) werden mit 293 mg (2,12 mmol) K₂CO₃, 106 ml (1,06 mmol) Dimethyldicarbonat und 53 mg DMAP in 20 ml Diethylenglycoldimethylether 2 Stunden am Rückfluß gekocht. Es wird abrottiert, der Rückstand mit EE/KH₂PO₄-Lösun g versetzt, die organische Phase abgetrennt und nach Trocknung über Na₂SO₄ eingeengt. Chromatographie an SiO₂ (EE/Heptan 2:1) liefert 225 mg der Titelverbindung.
mp.: 146°C,
Rf (SiO₂, EE) = 0,37,
MS (FAB): 532 (M+H)

### Beispiel 19

1-[(2'-Methoxycarbonylaminosulfonyl-biphenyl-4-yl)methyl]-2-n-propyl-4-methylthio-imidazol-5-carbonsäure

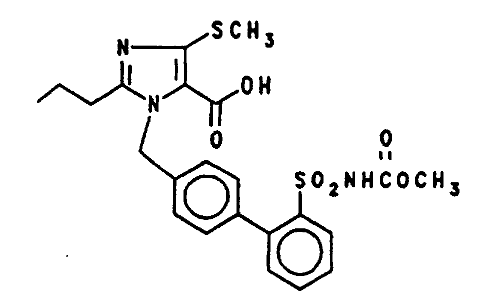

Die Versetzung von 150 mg (0,263 mmol) der Verbindung aus Beispiel 18) analog zu der des Beispiels 2) liefert 110 mg der Titelverbindung.
mp.: 131°C,
Rf (SiO₂, CH₂Cl₂/MCOH 10:1) = 0,15,
MS (FAB): 504 (M + H)

### Beispiel 20

1-[(2'-Cyclopropylmethylaminocarbonylaminosulfonyl-biphenyl-4-yl)methyl]-2-npropyl-4-methylthio-imidazol-5-carbonsäureethylester 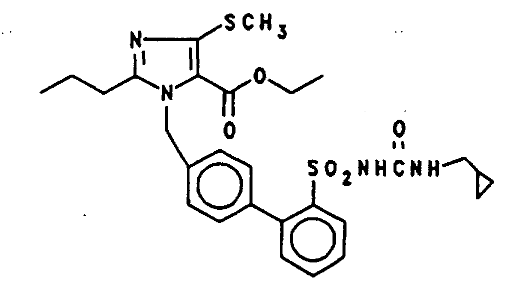 500 mg (1,06 mmol) der Verbindung aus Beispiel 1 j) werden mit 408 mg (1,06 mmol) Dihydroxybenzotriazolylcarbonat (70%ig) und 85 ml (1,06 mmol) Pyridin in 20 ml abs.l CH₂Cl₂ 2 Stunden bei Raumtemperatur gerührt. Die Reaktionslösung wird anschließend mit 114 mg (1,06 mmol) Cyclopropylmethylamin-hydrochlorid und 170 ml (2,12 mmol) Pyridin erneut für 2 Stunden gerührt. Es wird einrottiert, der Rückstand in EE aufgenommen, die EE-Phase mit NaHCO₃-, NaHSO₄-Lösung gewaschen, über Na₂SO₄ getrocknet und eingeengt. Chromatographie an SiO₂ (EE/Heptan 1:3) liefert 72 mg der Titelverbindung.
mp.: 125°C,
Rf (SiO₂, EE) = 0,47,
MS (FAB): 571 (M + H)

### Beispiel 21

1-[(2'-Cyclopropylmethylaminocarbonylaminosulfonyl-biphenyl-4-yl)methyl]-2-npropyl-4-methylthio-imidazol-5-carbonsäure

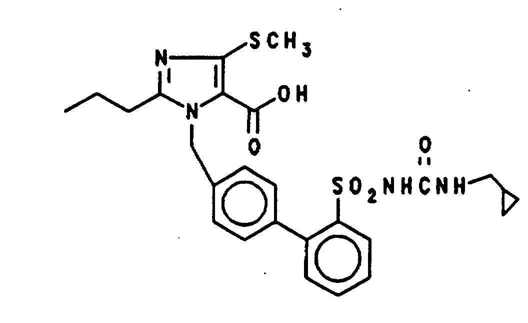

Die zum Beispiel 2) analoge Verseifung von 45 mg (0,08 mmol) der Verbindung aus Beispiel 20) liefert 34 mg der Titelverbindung.
mp.: 138°C,
Rf (SiO₂, SiO₂, CH₂Cl₂/MCOH 9:1) = 0,1,
MS (FAB): 543 (M+H)

### Beispiel 22

1-[(2'-Propyloxycarbonylaminosulfonyl-biphenyl-4-yl)methyl]-2-n-propyl-4-methylthioimidazol-5-carbonsäureethylester

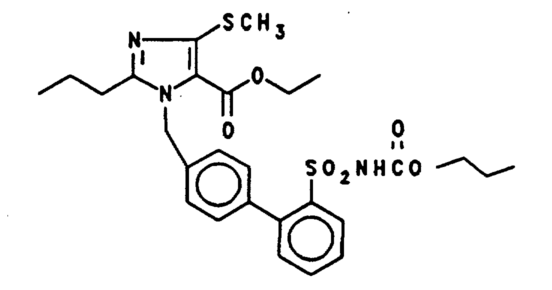

Der zum Beispiel 3) analoge Umsatz von 200 mg (0,42 mmol) der Verbindung aus Beispiel 1 j) mit 70 ml (0,63 mmol) Chlorameisensäurepropylester liefert nach Chromatographie an SiO₂ (EE/Heptan 2:1) 200 mg der Titelverbindung.
mp.: 144°C,
Rf (SiO₂, EE) = 0,54,
MS (FAB): 560 (M+H)

### Beispiel 23

1-[(2'-Propyloxycarbonylaminosulfonyl-biphenyl-4-yl)methyl]-2-n-propyl-4-methylthioimidazol-5-carbonsäure

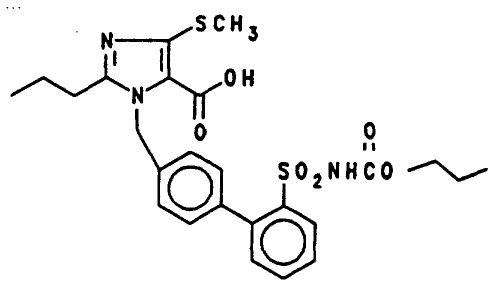

Diese Verbindung resultiert aus der Verbindung des Beispiels 22) nach dem Verfahren des Beispiels 2).
mp.: 124°C,
Rf (SiO₂, CH₂Cl₂/MCOH 10:1) = 0,1,
MS (FAB): 532 (M + H)

### Beispiel 24

1-[(2'-Benzyloxycarbonylaminosulfonyl-biphenyl-4-yl)methyl]-2-n-propyl-4-methylthioimidazol-5-carbonsäureethylester

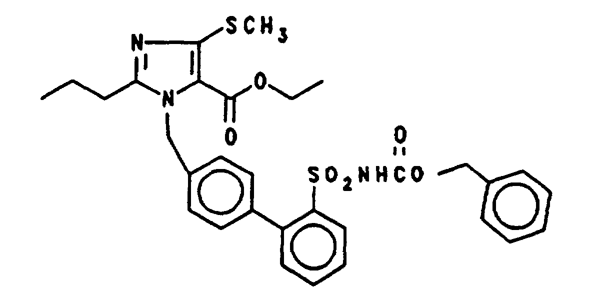

Der zum Beispiel 3) analoge Umsatz von 200 mg (0,42 mmol) der Verbindung aus Beispiel 1 j) mit 89 µl (0,633 mmol) Chlorameisensäurebenzylester liefert nach Chromatographie an SiO₂ (EE/Heptan 2:1) 250 mg der Titelverbindung.
mp.: 158°C,
Rf (SiO₂, EE) = 0,55,
MS (FAB): 608 (M + H)

### Beispiel 25

1-[(2'-Benzyloxycarbonylaminosulfonyl-biphenyl-4-yl)methyl]-2-n-propyl-4-methylthioimidazol-5-carbonsäure

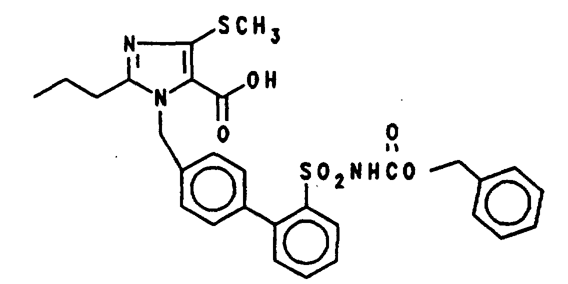

Diese Verbindung resultiert aus der Verbindung des Beispiels 24) nach dem Verfahren des Beispiels 2).
mp.: 115°C,
Rf (SiO₂, CH₂Cl₂/MCOH 15:1) = 0,1
MS (FAB): 580 (M + H)

## Patentansprüche

1. Verbindung der Formel (I) 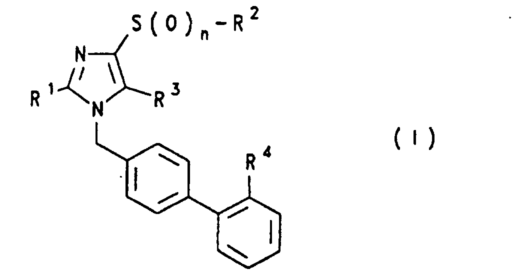 in welcher die Symbole folgende Bedeutung haben:
a)
R¹ (C₁-C₃)-Alkyl
b) R²
1. (C₁-C₆)-Alkyl
c) R³
1. CO-R⁶ oder
d) R⁴
1. SO₂-NR⁸-CO-NR⁷R⁹,
2. SO₂-NH-COO-R⁷,
3. SO₂-NH-CO-R⁷
e) R⁶
1. Wasserstoff oder
2. OR⁷
f)
R⁷,R⁹ gleich oder verschieden
1. Wasserstoff,
2. (C₁-C₆)-Alkyl
3. (C₃-C₆)-Cycloalkyl-(C₁-C₃)-alkyl,
4. (C₆-C₁₀)-Aryl-(C₁-C₄)-alkyl,
5. (C₂-C₆)-Alkenyl
g)
R⁸ Wasserstoff;
h)
n 0, 1 oder 2;
sowie deren physiologisch verträglichen Salze.

2. Verbindung gemäß Anspruch 1, **dadurch gekennzeichnet, daß** in Formel (I) R¹ Ethyl- oder n-Propyl bedeutet, oder deren physiologisch verträglichen Salze.

3. Verbindung gemäß Anspruch 1, **dadurch gekennzeichnet, daß** man in Formel (I)
R¹ für n-Propyl steht, oder deren physiologisch verträglichen Salze.

4. Verbindung gemäß einem der Ansprüche 1 bis 3, **dadurch gekennzeichnet, daß** in Formel (I)
R² (C₁-C₆)-Alkyl bedeutet,
R³ COR⁶ bedeutet,
n gleich Null ist,
R⁴ SO₂-NH-CO-OR⁷, SO₂NHCO-NHR⁷ oder SO₂-NH-CO-R⁷ bedeutet,
R⁶ Wasserstoff oder OR⁷ ist, und
R⁷ gleich Wasserstoff oder (C₁-C₆)-Alkyl bedeutet, sowie deren physiologisch verträglichen Salze.

5. Verfahren zur Herstellung einer Verbindung der Formel (I) gemäß Anspruch 1, sowie von deren physiologisch verträglichen Salzen, **dadurch gekennzeichnet, daß** man eine Verbindung der Formel (II) 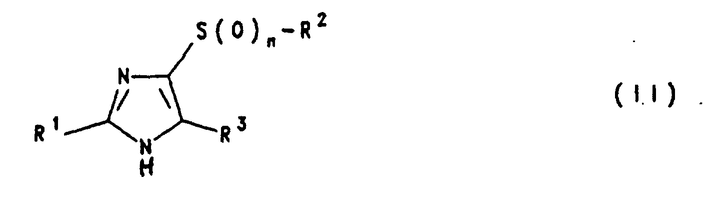 worin R¹, R², R³ und n wie in Anspruch 1 definiert sind, alkyliert mit Verbindungen der Formel (III) 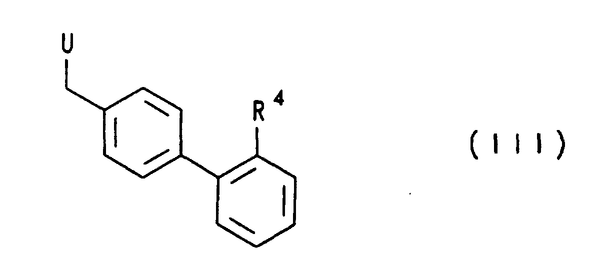 worin R⁴ wie in Anspruch 1 definiert sind, und U für eine Fluchtgruppe steht, gegebenenfalls temporär eingeführte Schutzgruppen wieder abspaltet, erhaltene Sulfonamide der Formel (I) gegebenenfalls in Urethane der Formel (I) überführt, erhaltene Sulfonamide der Formel (I) oder erhaltene Urethane der Formel (I) gegebenenfalls in Sulfonylharnstoffe der Formel (I) überführt, und die erhaltenen Verbindungen der Formel (I) gegebenenfalls in ihre physiologisch verträglichen Salze überführt.

6. Verbindung gemäß einem der Ansprüche 1 bis 4 zur Anwendung als Heilmittel.

7. Verbindung gemäß einem der Ansprüche 1 bis 4 zur Anwendung als Heilmittel zur Behandlung des Bluthochdrucks.

8. Pharmazeutische Zubereitung enthaltend eine Verbindung gemäß einem der Ansprüche 1 bis 4.

9. Verfahren zur Herstellung einer Zubereitung gemäß Anspruch 8, **dadurch gekennzeichnet, daß** man eine Verbindung der Formel (I) gemäß Anspruch 1 zusammen mit einem physiologisch unbedenklichen Träger und gegebenenfalls weiteren Zusatz- oder Hilfsstoffen in eine geeignete Darreichungsform bringt.

## Claims

1. A compound of the formula (I) 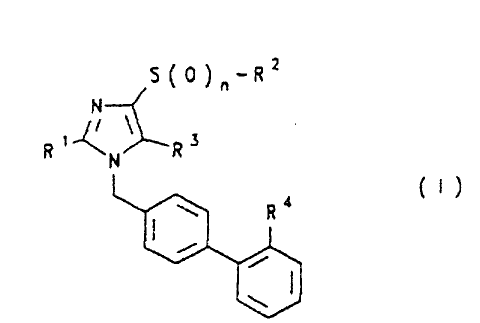 in which the symbols have the following meaning:
a) R¹ is (C₁-C₃)-alkyl;
b) R² is 1. (C₁-C₆)-alkyl;
c) R³ is 1. CO-R⁶;
d) R⁴ is
1. SO₂-NR⁸-CO-NR⁷R⁹,
2. SO₂-NH-COO-R⁷,
3. SO₂-NH-CO-R⁷;
e) R⁶ is
1. hydrogen or
2. OR⁷ ;
f) R⁷ and R⁹ are identical or different and are
1. hydrogen,
2. (C₁-C₆)-alkyl,
3. (C₃-C₆)-cycloalkyl-(C₁-C₃)-alkyl,
4. (C₆-C₁₀)-aryl-(C₁-C₄)-alkyl,
5. (C₂-C₆)-alkenyl;
g) R⁸ is hydrogen;
h) n is 0, 1 or 2;
and its physiologically tolerable salts.

2. A compound as claimed in claim 1, wherein in formula (I)
R¹ is ethyl or n-propyl, or its physiologically tolerable salts.

3. A compound as claimed in claim 1, wherein in formula (I)
R¹ is n-propyl, or its physiologically tolerable salts.

4. A compound as claimed in one of claims 1 to 3, wherein in formula (I)
R² is (C₁-C₆)-alkyl,
R³ is COR⁶,
n is equal to zero,
R⁴ is SO₂-NH-CO-OR⁷, SO₂NHCO-NHR⁷ or SO₂-NH-CO-R⁷,
R⁶ is hydrogen or OR⁷, and
R⁷ is equal to hydrogen or (C₁-C₆)-alkyl, and their physiologically tolerable salts.

5. A process for the preparation of a compound of the formula (I) as claimed in claim 1, and of its physiologically tolerable salts, which comprises alkylating a compound of the formula (II) 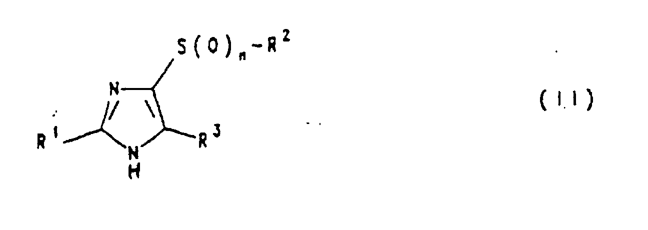 in which R¹, R², R³ and n are as defined in claim 1, with a compound of the formula (III) 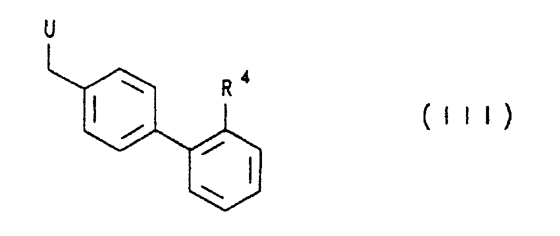 in which R⁴ is as defined in claim 1, and U is a leaving group, optionally removing temporarily introduced protective groups again, optionally converting sulfonamides of the formula (I) obtained into urethanes of the formula (I), optionally converting sulfonamides of the formula (I) obtained or urethanes of the formula (I) obtained into sulfonylureas of the formula (I) and optionally converting the compounds of the formula (I) obtained into their physiologically tolerable salts.

6. A compound as claimed in one of claims 1 to 4 for use as a medicine.

7. A compound as claimed in one of claims 1 to 4 for use as a medicine in the treatment of high blood pressure.

8. A pharmaceutical preparation comprising a compound as claimed in one of claims 1 to 4.

9. A process for the production of a composition as claimed in claim 8, which comprises bringing a compound of the formula (I) as claimed in claim 1 into a suitable administration form together with a physiologically acceptable excipient and, if appropriate, other additives or auxiliaries.

## Revendications

1. Composé de formule (I) 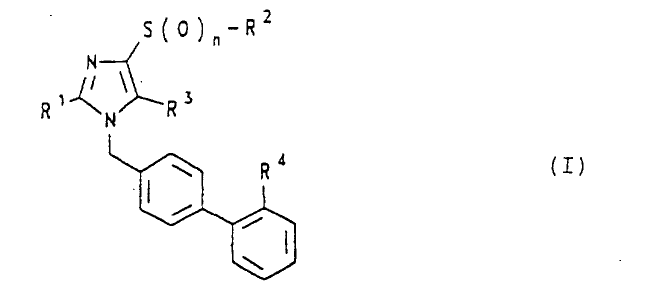 où les symboles possèdent la signification suivante :
a) R¹ représente un groupe alkyle en C₁-C₃,
b) R² représente 1. un groupe alkyle en C₁-C₆ ;
c) R³ représente 1. un groupe CO-R⁶ ;
d) R⁴ représente
1. un groupe SO₂-NR⁸-CO-NR⁷R⁹,
2. un groupe SO₂-NH-COO-R⁷,
3. un groupe SO₂-NH-CO-R⁷ ;
e) R⁶ représente
1. un atome d'hydrogène ou
2. un groupe OR⁷ ;
f) R⁷, R⁹ sont identiques ou différents et représentent
1. un atome d'hydrogène,
2. un groupe alkyle en C₁-C₆,
3. un groupe (cycloalkyle en C₃-C₆)-alkyle en C₁-C₃,
4. un groupe (aryle en C₆-C₁₀)-alkyle
5. un groupe alcényle en C₂-C₆,
g) R⁸ représente un atome d'hydrogène ;
h) n vaut 0, 1 ou 2 ;
ainsi que leurs sels tolérés du point de vue physiologique.

2. Composé selon la revendication 1, **caractérisé en ce que** à la formule (I) R¹ représente un groupe éthyle ou propyle, ou leurs sels tolérés du point de vue physiologique.

3. Composé selon la revendication 1, **caractérisé en ce que** à la formule (I), R¹ représente un groupe propyle ou ses sels tolérés du point de vue physiologique.

4. Composé selon l'une des revendications 1 à 3, **caractérisé en ce que** à la formule (I)
R² représente un groupe alkyle en C₁-C₆,
R³ représente un groupe CO-R⁶,
n est égal à zéro,
R⁴ des groupes SO₂-NH-CO-OR⁷, SO₂-NHCO-NHR⁷ ou SO₂-NH-CO-R⁷,
R⁶ représente un atome d'hydrogène ou un groupe OR⁷, et
R⁷ est identique à un atome d'hydrogène ou un groupe alkyle en C₁-C₆, ainsi que ses sels tolérés du point de vue physiologique.

5. Procédé pour la préparation d'un composé de formule (I) selon la revendication 1, ainsi que de ses sels tolérés du point de vue physiologique, **caractérisé en ce qu'**on alkyle un composé de formule (II) 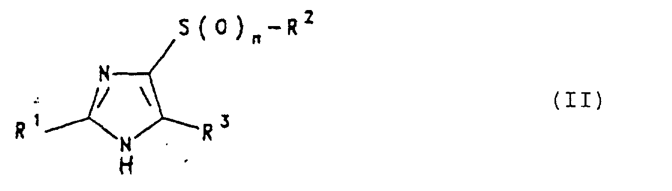 où R¹, R², R³ et n sont définis à la revendication 1, par des composés de formule (III) 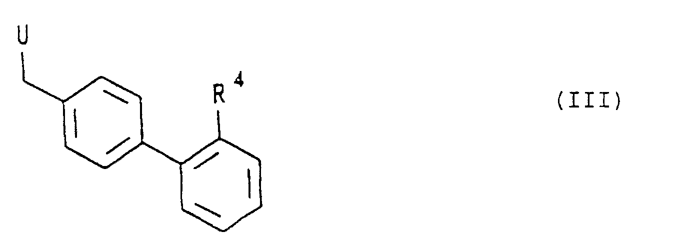 où R⁴ est défini comme ci-dessus et U est un groupe fuyant, on sépare à nouveau des groupes protecteurs éventuellement temporairement introduits, on transforme des sulfonamides de formule (I) obtenus éventuellement en uréthannes de formule (I), on transforme des sulfonamides de formule (I) obtenus ou des uréthannes de formule (I) obtenus éventuellement en sulfonylurées de formule (I), et on transforme les composés de formule (I) obtenus éventuellement en leurs sels tolérés du point de vue physiologique.

6. Composé selon l'une des revendications 1 à 4 pour l'utilisation comme médicament.

7. Composé selon l'une des revendications 1 à 4 pour l'utilisation comme médicament pour le traitement de l'hypertension.

8. Préparation pharmaceutique contenant un composé selon l'une des revendications 1 à 4.

9. Procédé pour la préparation d'une préparation selon la revendication 8, **caractérisé en ce qu'**on met en forme d'administration appropriée un composé de formule (I) selon la revendication 1, conjointement avec des véhicules inoffensifs du point de vue physiologique et éventuellement avec d'autres matières d'addition ou adjuvants.
